# EUROPEAN PATENT APPLICATION

(11) **EP 2 461 161 A1**
(43) Date of publication of application: **06.06.2012**
(21) Application number: 10804129.4
(22) Date of filing: 29.07.2010
(51) Int. Cl.: G01N 33/543, G01N 33/532

(54) **ALLOYED METAL COLLOID**

(30) Priority: 29.07.2009 JP 2009176812
(71) Applicant: Kogakuin University, Tokyo 163-8677 (JP); Otsuka Pharmaceutical Co., Ltd., Chiyoda-ku Tokyo 101-8535 (JP)
(72) Inventor: WATABE, Masatoshi, Hachioji-shi Tokyo 193-0802 (JP); ODA, Tetsuya, Osaka-shi Osaka 541-0045 (JP); AKAMATSU, Suguru, Osaka-shi Osaka 541-0045 (JP)
(74) Representative: Blodig, Wolfgang
(86) International application number: PCT/JP2010/004816
(87) International publication number: WO 2011/013378

(57) **Abstract**

Provided is a metal colloid having higher visibility and higher sensitivity than a gold colloid and a Au-core Pt-shell composite colloid and suitable as a labeling agent for use in a test such as an immunoassay. An alloyed Au/Pt composite colloid formed by mixing a gold salt and a platinum salt with at least one reducing agent selected from the group consisting of an amino acid and a derivative thereof, an oligopeptide and a derivative thereof, and an amino sugar in the presence of an alkali, thereby reducing the gold salt and platinum salt.

## Description

### [Technical Field]

The present invention relates to an alloyed metal colloid suitable as a labeling agent for use in a test, etc., and a production method thereof.

### [Background Art]

In the development of test drugs, a quantitative reagent and a qualitative reagent are separately produced depending upon clinical purposes. In detecting an infective antigen such as an influenza virus as well as a myocardial marker etc., a rapid diagnostic test such as a POCT is of importance. Thus, immunochromatography capable of detecting an antigen easily in a short time is employed.

In the immunochromatography, colored latex, metal colloid, an enzymatic label, or the like is used as a colored probe for labeling an antibody which can be used for visual detection. Of these, metal colloid is most commonly used since it is relatively easily handled and develops a highly intensive visible color compared to a latex particle.

The metal colloid most frequently used is a gold (Au) colloid prepared by citrate reduction. However, the sensitivity of immunochromatography using a gold colloid label is not always sufficient. In addition, the gold colloid is only red to red purple, and thus a problem thereof is that two-color determination as for latex cannot be made.

As another metal colloid, a platinum (Pt) colloid is known to appear black. Since a nitrocellulose membrane used in immunochromatography is white, if a black colloid can be used, improvement in visibility due to high contrast difference between black and white and further improvement in sensitivity are expected.

Conventionally, it has been reported that a platinum colloid is synthesized by citrate reduction or by using a stronger reducing agent and a protective agent containing a polymer compound and recently that a platinum colloid is synthesized by using a protective agent containing a low molecular weight compound (Patent Document 1, Patent Document 2). However, a platinum colloid synthesized by any of the above synthetic methods has a small particle size. Thus, the platinum colloid is lower in visibility than the gold colloid having a larger particle size if the same number of particles are used. Therefore, the platinum colloid is impractical.

As platinum colloids having a large particle size, composite colloids have been reported (Non Patent Documents 1 to 7) including composite colloids, for example, having a Au-core Pt-shell structure in which platinum as a shell is supported on the surface of a gold colloid as a core, a Pt-core Au-shell structure and an alloy structure in which Au and Pt are integrated.
Furthermore, it has also been reported that if a Au-core Pt-shell composite colloid is used in immunochromatography, the sensitivity of measurement increases (Patent Document 3); however, the sensitivity cannot be said to be sufficient.

### [Prior Art Documents]

### [Patent Documents]

[Patent Document 1] JP-A-2002-239372
[Patent Document 2] WO 2005/023468
[Patent Document 3] JP-B-3886000

### [Non Patent Documents]

[Non Patent Document 1] R. S. Minor et al., 7th International Congress on catalysis. Elsevier S. P. Co. 1981.
[Non Patent Document 2] P. A. Sermon et al., Angew. Chem. Int. Ed. Engl. 26(1987) No.9.
[Non Patent Document 3] P.A. Sermon et al., Mat. Res. Soc. Symp. Proc. Vol. 1988 Materials Research Society.
[Non Patent Document 4] G. Schmid et al., Angew. Chem. Int. Ed. Engl. 30(1991) No.7.
[Non Patent Document 5] T. Yonezawa et al., J. Molecular Catalysis 83(1993) 167.
[Non Patent Document 6] T. Yonezawa et.al., J.C.S. Faraday Trans., 1995, 91(22), 4111.
[Non Patent Document 7] A. Henglein J. Phys. Chem. B2000, 104, 2201.

### [Summary of the Invention]

### [Problems to be Solved by the Invention]

The present invention relates to providing a metal colloid having higher visibility and higher sensitivity than a gold colloid and a Au-core Pt-shell composite colloid and suitable as a labeling agent for use in a test such as an immunoassay.

### [Means for Solving the Problems]

The present inventors have extensively studied on a Au/Pt composite colloid excellent in visibility. As a result, they have found that if a colloid to be a core is not prepared but a gold salt and a platinum salt are mixed, in advance, with a reducing agent such as an amino acid and a peptide to prepare a colloid, then a stable alloyed Au/Pt composite colloid particle can be obtained, and further found that since the colloid has high visibility, if the colloid is used as a labeling agent for an immunoassay, the colloid provides a high signal intensity and enables more sensitive measurement.

Accordingly, the present invention relates to the following items 1) to 4):
1) An alloyed Au/Pt composite colloid formed by mixing a gold salt and a platinum salt with at least one reducing agent selected from the group consisting of an amino acid and a derivative thereof, an oligopeptide and a derivative thereof, and an amino sugar in the presence of an alkali, thereby reducing the gold salt and platinum salt.
2) The alloyed Au/Pt composite colloid according to item 1), wherein the gold salt and platinum salt are used in such a way that an amount of Pt is 7 to 25% of an amount of Au.
3) A labeling agent for an immunoassay containing the alloyed Au/Pt composite colloid according to item 1) or 2).
4) An antibody or antigen labeled with the alloyed Au/Pt composite colloid according to item 1) or 2).
5) A method for producing an alloyed Au/Pt composite colloid including mixing a gold salt and a platinum salt with at least one reducing agent selected from the group consisting of an amino acid and a derivative thereof, an oligopeptide and a derivative thereof, and an amino sugar in the presence of an alkali, thereby reducing the gold salt and platinum salt.

### [Effects of the Invention]

The alloyed Au/Pt composite colloid of the present invention has higher visibility and higher sensitivity than a gold colloid and a Au-core Pt-shell composite colloid, and thus extremely useful, for example, as a labeling agent for use in a test such as an immunoassay.

### [Brief Description of the Drawings]

[Figure 1] Figure 1 is an electron micrograph of an alloyed Au/Pt composite colloid (200,000 magnifications, 1 graduation = 8 nm).
[Figure 2] Figure 2 is the results (a photograph of a determination part) of immunochromatographic measurement using an alloyed Au/Pt composite colloid. C: control line, T: test line
[Figure 3] Figure 3 is the results (photograph of a determination part) of immunochromatographic measurement using alloyed Au/Pt composite colloids synthesized by varying the amount of Pt. C: control line, T: test line [Figure 4] Figure 4 is the results (signal intensity of a test line) of immunochromatographic measurement using alloyed Au/Pt composite colloids synthesized by varying the amount of Pt.
[Figure 5] Figure 5 is the results (color tone) of immunochromatographic measurement using alloyed Au/Pt composite colloids synthesized by varying the amount of Pt.
[Figure 6] Figure 6 is the results (photograph of a determination part) of immunochromatographic measurement using a Au-core Pt-shell Au/Pt composite colloid. C: control line, T: test line

### [Modes for Carrying out the Invention]

In the present invention, examples of the "gold salt" used herein include chloroauric acid, gold(I) potassium cyanide, gold(III) potassium cyanide, tetraamminegold nitrate, tetranitratogold ammonium salt and diaqua(1,10-phenanthroline)gold nitrate. Of these, chloroauric acid is preferred.
Furthermore, examples of the "platinum salt" include hexachloroplatinic acid, dinitrodiammineplatinum, dinitrodiammineplatinum nitrate, *cis-*diamminediaquaplatinum nitrate, trans-diamminediaquaplatinum nitrate, tetranitroplatinic acid, tetra(oxalato)platinic acid, *cis*-dinitrodiaquaplatinum, tetraammineplatinum hydroxide, hexaamineplatinum hydroxide, tetraamineplatinum chloride, hexaamineplatinum chloride, hexahydroxyplatinic acid, platinum oxide, platinum(II) chloride, platinum (IV) chloride and potassium tetrachloroplatinate. Of these, potassium tetrachloroplatinate, hexachloroplatinic acid and the like are preferred.

In the present invention, examples of the reducing agent include an amino acid, an oligopeptide and an amino sugar. They exhibit no reduction property in a normal environment; however, exhibit a reduction property for reducing a gold ion and a platinum ion in an alkaline environment.

Examples of the amino acid include α-amino acids such as glycine, alanine, serine, valine, aspartic acid, asparagine, glutamic acid, glutamine, isoleucine, leucine, lysine, arginine, cysteine, methionine, proline, threonine, phenylalanine, tyrosine, tryptophan, arginine and histidine. Examples of its derivative include a carboxylic acid ester derivative (such as methyl ester and ethyl ester) and a carboxylic acid amide derivative.

Examples of the oligopeptide include dipeptides such as glycylglycine, glycylalanine and alanylalanine and tripeptides such as glycylglycylglycine and glycylglycylalanine. An oligopeptide containing another amino acid may be used. Examples of these derivatives include a carboxylic acid ester derivative (such as methyl ester and ethyl ester) and a carboxylic acid amide derivative. For example, as an ester derivative of a dipeptide residue, aspartame (N-(L-α-aspartyl)-L-phenylalanine 1-methylester) etc., can be mentioned.

Examples of the amino sugar include glucosamine, N-acetylglucosamine, galactosamine and N-acetylgalactosamine.

Of these reducing agents, preferable examples include an amino acid such as glycine and alanine and an oligopeptide such as glycylglycine.
The reducing agents such as an amino acid and a derivative thereof, an oligopeptide and a derivative thereof and an amino sugar may be used singly or in combinations thereof.
Note that the amino acid, oligopeptide, amino sugar, etc. to be used as a reducing agent in the present invention surrounds an alloyed Au/Pt composite particle and functions as a protective component. Accordingly, the colloid of the present invention can be stably present even if a protective component is not separately added since the alloyed Au/Pt composite particles formed are dispersed without precipitating. However, a sulfur-containing amino acid such as methionine and cysteine and gelatin colloid may be added to further prevent precipitation.

Examples of the alkali include an alkali metal hydroxide such as sodium hydroxide and potassium hydroxide, an alkali metal carbonate such as sodium carbonate and potassium carbonate, an alkaline-earth metal hydroxide such as magnesium hydroxide and calcium hydroxide; an alkaline-earth metal carbonate such as magnesium carbonate and calcium carbonate, hydrazine, hydroxyl amine and ammonia. An alkaline metal hydroxide such as sodium hydroxide and potassium hydroxide is preferred.

Preparation of the alloyed Au/Pt composite colloid of the present invention will be described below.

### 1) Formation of alloyed Au/Pt composite particle (colloid)

With a solvent, a gold salt, a platinum salt and a reducing agent are mixed in the presence of an alkali and reacted to obtain an alloyed Au/Pt composite particle (colloid).
As the solvent used herein, water is usually used; however, ultrapure water, and ion-exchanged water distillated several times can be used.
Note that a method of adding a gold salt, a platinum salt and a reducing agent is not particularly limited. For example, a method of adding a gold salt to a solvent, then adding a reducing agent and subsequently adding a platinum salt is mentioned.

It is desirable that the amount of gold salt added be preferably 0.01 to 0.4 w/v% based on the total amount of solvent and more preferably 0.05 to 0.09 w/v%.
Furthermore, in view of enhancing visibility of the produced Au/Pt composite colloid, particularly in view of enhancing a signal intensity (detection sensitivity) when the colloid is used as a labeling substance (colored particle) for immunochromatography, it is desirable that the amounts of gold salt and platinum salt added be controlled in such a way that the amount of Pt is 7 to 25% and more preferably 12 to 18% of the amount of Au.

The amount of a reducing agent added varies depending upon the type thereof; however, it is preferably 1 to 12 moles relative to a gold salt and platinum salt (1 mole) and more preferably 4 to 6 moles.

An alkali is usually used within the range of 5 mmol/L to 30 mmol/L, and preferably 12 mmol/L to 14 mmol/L in terms of concentration in a solution, and desirably added so that the pH of the solution is 10 or more and preferably 11 to 12.

The reaction is usually performed at 90 to 98°C, preferably 93 to 97°C, for 10 to 60 minutes, preferably for 20 to 45 minutes under stirring. The reaction is preferably performed at 40 to 65°C for 5 to 20 minutes followed by raising the temperature (to 93 to 98°C) and performing the reaction for 20 to 60 minutes.
Through the reaction mentioned above, an alloyed Au/Pt composite particle (colloid) can be obtained.

### 2) Unreacted substance and desalting treatment

Subsequently, the obtained alloyed Au/Pt composite particle (colloid) is treated for lowering the contents of an unreacted substance and a salt.
Examples of such a treatment include (1) ultrafiltration using a centrifuge (a filter with a molecular weight cutoff of 30000 is used and centrifugation is performed at 2000 to 5000 rpm for 30 minutes) followed by appropriate addition of water, (2) use of permeable membrane, and (3) separation by an ultracentrifuge (6000 rpm, for 10 minutes) followed by appropriate addition of water.

### 3) Neutralization treatment

Subsequently, if necessary, a neutralization treatment is performed in such a way that the pH of a solution is 7 to 9.
The neutralization treatment is performed by, for example, centrifuging a colloid solution to separate a precipitate and a supernatant, removing the supernatant to recover the precipitate, diluting the precipitate with water, and neutralizing in such a way that the pH is not reduced to 6 or less. The concentration of the colloid can be controlled depending upon the amount of water added herein and the concentration can be easily increased.

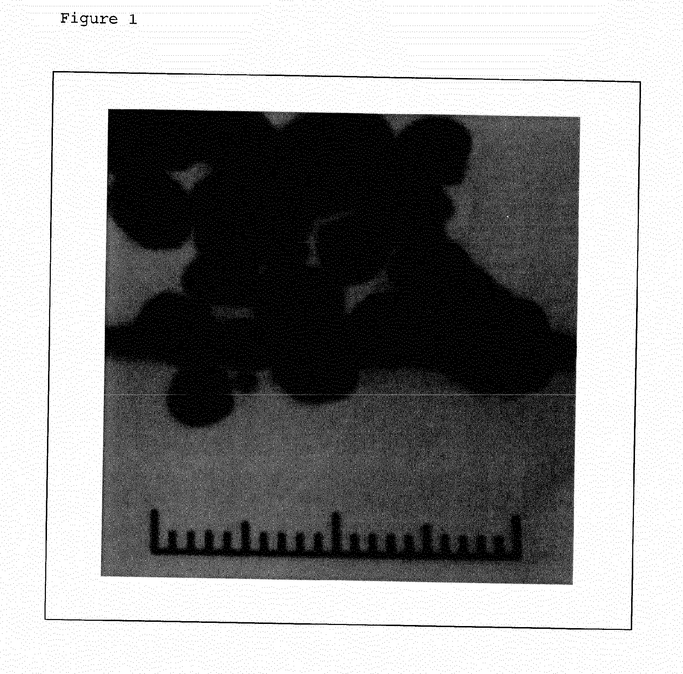

The colloid thus obtained presents black purple close to black. The colloid is so-called dispersed colloid in which the particles are stably present while being dispersed without precipitating. According to electron microscopic observation, the colloid is not a Au-core Pt-shell colloid but a smooth alloyed (homogeneous alloy) Au/Pt composite colloid where Au and Pt are integrated (see Figure 1, 1 graduate = 8 nm). The average particle size of the Au/Pt composite particle is 20 nm to 100 nm. Note that the average particle size is obtained, for example, by taking a photograph by a transmission electron microscope (TEM), measuring particle sizes of 100 particles and obtaining an average value thereof.
Therefore, the alloyed Au/Pt composite colloid can be suitably used as a labeling agent (visible probe) for immunochromatography and other various immunoassays.

A protein such as an antigen and an antibody can be labeled with the alloyed Au/Pt composite colloid of the present invention in accordance with a customary method. For example, an antibody is mixed with an alloyed Au/Pt composite colloid and the mixture is stirred for 20 minutes to 24 hours to bind the antibody to the colloid. Then, the surface of colloid is blocked by adding a casein solution and BSA etc. Subsequently, the supernatant is centrifugally removed and then the resultant precipitate is suspended in a buffer containing casein to obtain an alloyed Au/Pt composite colloid-labeled antibody.

### [Examples]

### Example 1 Synthesis of alloyed Au/Pt composite colloid

To water (253.2 ml), 1% (w/v) chloroauric acid (18 ml) was added, subsequently, 1% (w/v) glycine (18 ml) serving as a reducing agent, 0.5 N KOH (8.1 ml) and 1% (w/v) potassium chloroplatinate (II) (2.70 mL (amount of Pt 15%)) were added. The reaction solution was allowed to react at 50°C for 15 minutes. Furthermore, the reaction solution was transferred to a water bath of 98°C and the reaction was performed for 30 minutes. After water (300. ml) was added, the reaction solution was concentrated to 100 ml by ultrafiltration to obtain a Au/Pt composite colloid.
The colloid obtained by the method presents black purple close to black. According to electron microscopic observation, the colloid was not a Au-core Pt-shell colloid but a smooth alloyed (homogeneous alloy) Au/Pt composite colloid where Au and Pt are integrated. The electron micrograph is shown in Figure 1.

### Example 2 Preparation of labeled antibody

To the obtained alloyed Au/Pt composite colloid (1 mL) (OD₅₂₀ = 10), an anti-human IgG goat antibody (20 µg) was added, followed by stirring at room temperature for about 6 hours to bind the antibody to the colloid. A casein solution was added so as to obtain a final concentration of 1% and the resultant solution was stirred at room temperature overnight to block the colloid surface. Centrifugation was performed at 4500 g and 4°C for 10 minutes and the recovered product was suspended in a buffer containing 1% casein to prepare an alloyed Au/Pt composite colloid-labeled antibody.

### Test Example 1 Measurement by immunochromatography

The alloyed Au/Pt composite colloid-labeled antibody prepared in Example 2 was subjected to measurement by the following immunochromatography to evaluate the performance of the colloid as a visible probe.
1) As the immunochromatography for evaluation, a measurement system for detecting anti-*Helicobacter pylori* antibody in a specimen was used. In the immunochromatography, an immunochromatographic test strip formed of a nitrocellulose membrane onto which an antigen extracted from a *Helicobacter pylori* cell was immobilized is used; a specimen is reacted with the immunochromatographic test strip and an anti*-Helicobacter pylori* antibody bound to the antigen on the membrane is detected by a colloid-labeled anti-human IgG antibody. Methods of preparing materials and a measurement method will be described below.

### 2) Preparation of antigen-immobilized membrane

Onto a nitrocellulose membrane, an antigen extracted from a *Helicobacter pylori* cell was linearly applied in an amount of 3 µg/cm. Simultaneously, an appropriate amount of anti-human IgG goat antibody was linearly applied. After application, the nitrocellulose membrane was dried at 37°C for 2 hours or more and then blocking was performed by soaking the membrane in a buffer containing skim milk in order to prevent non-specific adsorption. Thereafter, the membrane was sufficiently dried to obtain an antigen-immobilized membrane.

### 3) Preparation of immunochromatographic test strip

A sample pad formed of a glass fiber, etc., and an absorption pad formed of cellulose, etc., were attached to the antigen-immobilized membrane prepared in the above 2). The sample pad was allowed to be in contact with the specimen to be measured, thereby supplying the specimen to the antigen-immobilized membrane. The absorption pad was used for absorbing the specimen spreading over the membrane. The membrane having pads attached thereon was cut into strips of 4 mm in width to obtain immunochromatographic test strips.

### 4) Measurement method

The serum taken from a *Helicobacter pylori*-positive subject was diluted with a buffer to obtain a positive control specimen as an anti-*Helicobacter pylori* antibody-positive specimen. Urine taken from a *Helicobacter* pylori-positive subject was used in measurement as an anti-*Helicobacter pylori* antibody weak-positive specimen. In addition, urine taken from a *Helicobacter pylori-*negative subject was used as an anti*-Helicobacter pylori* antibody-negative specimen. Procedures for measurement and determination will be described below.
i) Each specimen is diluted with a measurement buffer and added dropwise to a measurement container.
ii) Each antibody labeled with the alloyed Au/Pt composite colloid is added to the measurement container and mixed therein.
iii) To the measurement container, the immunochromatographic test strip is placed and allowed to stand still at room temperature for 15 minutes.
iv) Fifteen minutes later, the emergence of a color line is visually confirmed.
Note that, a portion on a test strip having the antigen extracted from *Helicobacter pylori* applied thereon is used as a test line portion; whereas a portion having the anti-human IgG antibody applied thereon is used as a control line portion. The case where a color line emerged on both the test line portion and the control line portion is determined as positive. The case where the color line emerged only in the control line portion is determined as negative.

### 5) Measurement results

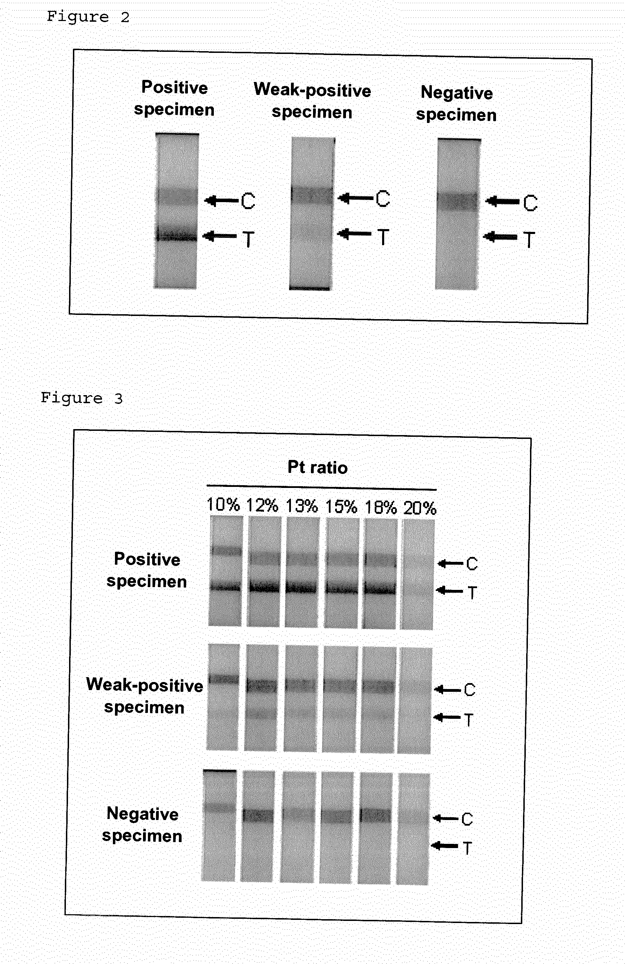

The measurement results are shown in Figure 2.
From Figure 2, the colloid was found to have good contrast and signal intensity, indicating that the colloid has high performance suitable for immunochromatography. It is demonstrated that the alloyed Au/Pt composite colloid prepared by the method of the present invention can be used as a high-performance visible probe for immunochromatography.

### Example 3

Au/Pt composite colloids were synthesized by varying the amount of Pt to be mixed with chloroauric acid to 10%, 12%, 13%, 15%, 18% and 20%. To water (253.2 ml), added were 1% (w/v) chloroauric acid (18 ml) and 1%(w/v) potassium chloroplatinate (II) in an amount of 1.80 mL (amount of Pt: 10%), 2.70 mL (amount of Pt: 15%), 2.16 mL (amount of Pt: 12%), 2.34 mL (amount of Pt: 13%), 2.70 mL (amount of Pt: 15%), 3.24 mL (amount of Pt: 18%) or 3.60 mL (amount of Pt: 20%). Subsequently, 0.5 N KOH (8.1 ml) and 1%(w/v) glycine (18 ml) serving as a reducing agent were added and thereafter each reaction solution was allowed to react at 50°C for 15 minutes. Furthermore, the reaction solution was transferred to a water bath of 98°C and the reaction was performed for 30 minutes. After water (300 ml) was added, the reaction solution was concentrated to 100 ml by ultrafiltration. In this manner, Au/Pt composite colloids were prepared, which contained Pt in amounts of 10%, 12%, 13%, 15%, 18% and 20%, respectively.
Using each of the alloyed Au/Pt composite colloids thus obtained, an alloyed Au/Pt composite colloid-labeled antibody was prepared in the same manner as in Example 2.

### Test Example 2

Using the alloyed Au/Pt composite colloid-labeled antibody prepared in Example 3, immunochromatographic measurement (Figure 3) was performed in the same manner as in Test Example 1 to evaluate the performance of the colloid as a visible probe.

### 1) Signal intensity

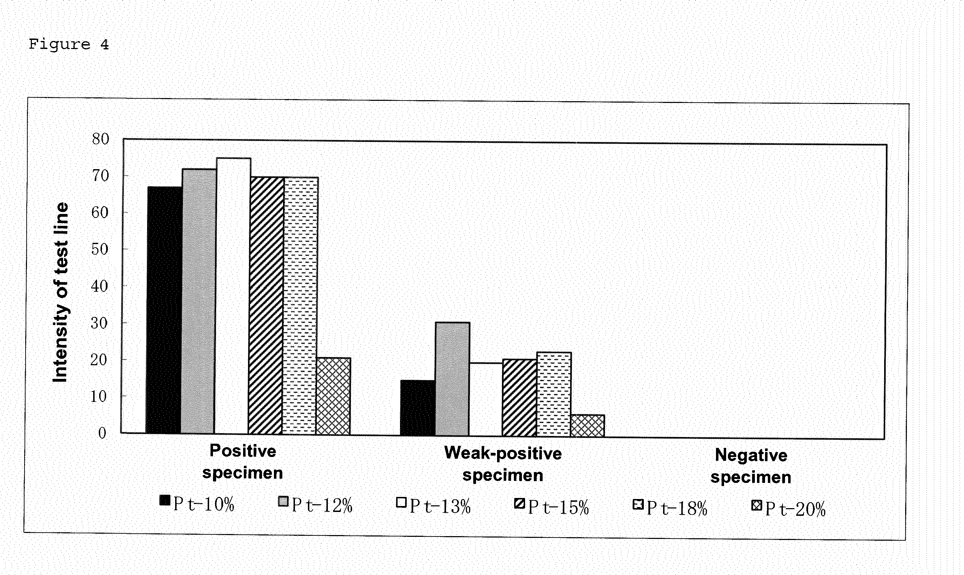

After completion of the measurement, a photograph of a determination part of the test strip was taken. The test line in the photograph was analyzed by image analysis software (densito graph, ATTO Corporation). The intensity of emerged lines was converted into numeral values indicating signal intensity and comparison was made (Figure 4).
The signal intensity of the antibodies labeled with the alloyed Au/Pt composite colloids prepared by varying the amount of Pt was weak in the case of an amount of Pt of 20%; however in the cases of an amount of Pt between 10 to 18%, no significant difference was observed and a weak-positive specimen was also clearly confirmed.

### 2) Color tone

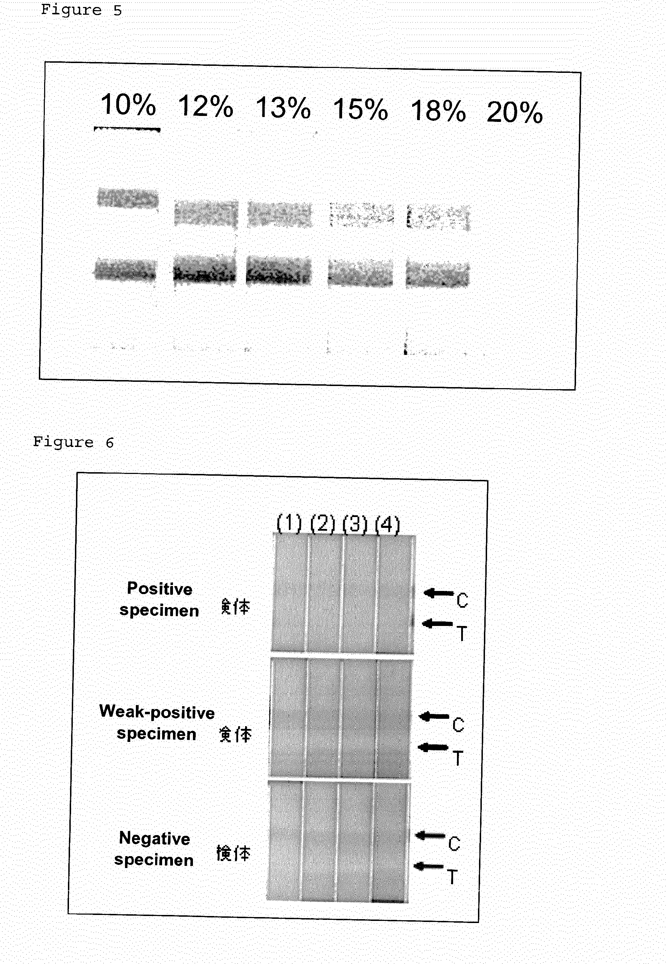

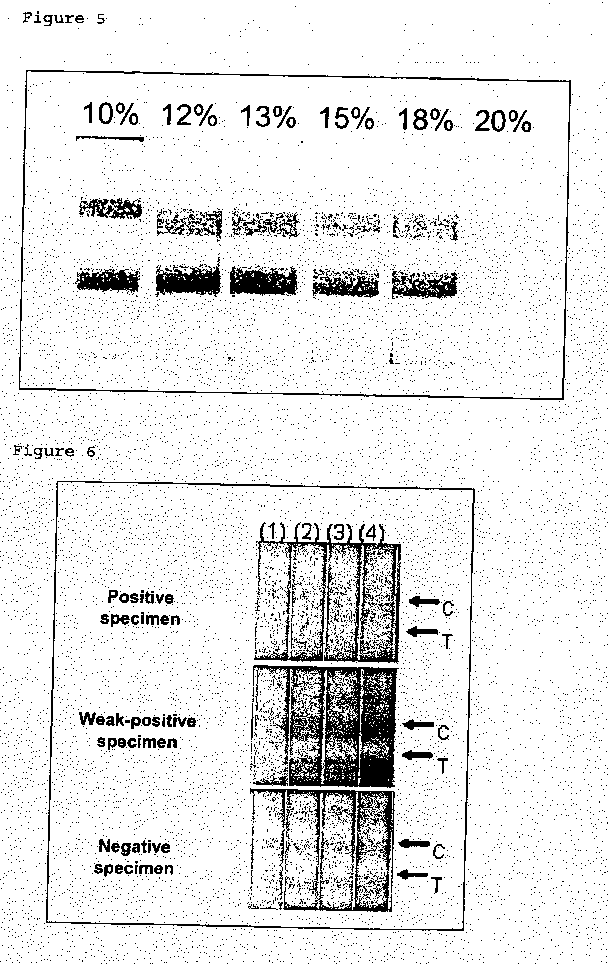

The photograph of measurement results of the positive specimen taken in the above 1) was controlled in brightness, contrast, etc., by image software (Photoshop (registered trademark), Adobe Systems Incorporated). As a result, the color tone of the composite colloids having an amount of Pt of 12 to 18% was close to black; however, the color tone of the composite colloid having an amount of Pt of 10% was strong red (Figure 5). Since color tone close to black rather than red is expected to show high contrast to white nitrocellulose membrane, the alloyed Au/Pt composite colloids having an amount of Pt of 12 to 18% are preferred as a visible probe.

### Comparative Example

Using a method described in Patent Document 3 (JP-B-3886000), a Au-core Pt-shell Au/Pt composite colloid was synthesized. Immunochromatographic measurement was performed in the same manner as in Test Example 1 to evaluate the performance of the colloid as a visible probe.

### (1) Synthesis of colloid

Ultrapure water (390 ml) was poured in a flask and boiled. To the boiled water, 30 ml of an aqueous chloroauric acid solution (containing 1 g of gold in 1L of solution) was added, and then, a 1 wt% aqueous sodium citrate solution (60 ml) was added. Six minutes and 45 seconds later, 30 ml of an aqueous chloroplatinic acid solution (containing 1 g of platinum in 1L of solution) was added. Five minutes after addition of the aqueous chloroplatinic acid solution, the 1 wt% aqueous sodium citrate solution (60 ml) was added and the resultant solution was subjected to reflux for 4 hours to obtain a colloid. The obtained colloid (1 ml) was centrifuged at 13800 x g for 25 minutes. After the supernatant was removed, ultrapure water (0.5 ml) was added to the remaining precipitate and stirred. The precipitate was resuspended by ultrasonication and the pH of the suspension was adjusted to 9.0 with a 200 mM aqueous potassium carbonate solution. To the suspension, ultrapure water was added to a total volume of 100 ml to obtain a Au/Pt composite colloid. The colloid would have a Au-core Pt-shell structure where a core formed of Au colloid is entirely or partially coated with Pt.

### (2) Preparation of labeled antibody

To check the immunochromatographic performance of the colloid, an anti-human IgG goat antibody (6.8 µg) was mixed with the colloid (1 mL) (OD₅₂₀ = 3.4) obtained above and pH of the mixture was adjusted to be 7 to 10 in order to obtain optimal labeling conditions. The mixture was stirred at room temperature for about 6 hours to bind the antibody to the colloid. A casein solution was added to obtain a final concentration of 1% and the resultant solution was stirred at room temperature overnight to block the surface of the colloid. Centrifugation was performed at 4500 g and 4°C for 40 minutes and the recovered product was suspended in a buffer containing 1% casein to prepare a Au/Pt composite colloid-labeled antibody.

### (3) Immunochromatographic measurement

The Au/Pt composite colloid-labeled antibody prepared above was subjected to immunochromatographic measurement in the same manner as in Example 1.

### (4) Measurement results

As a result of the measurement, the signal intensity of a test line emerged in positive specimen measurement using each labeled antibody was extremely weak. Furthermore, in weak-positive specimen measurement, a test line was not observed. Thus, immunochromatographic performance was not sufficient. Immunochromatographic measurement results (photographs of determination parts having a control line and a test line on a test strip) are shown in Figure 6.

## Claims

1. An alloyed Au/Pt composite colloid formed by mixing a gold salt and a platinum salt with at least one reducing agent selected from the group consisting of an amino acid and a derivative thereof, an oligopeptide and a derivative thereof, and an amino sugar in the presence of an alkali, thereby reducing the gold salt and platinum salt.

2. The alloyed Au/Pt composite colloid according to claim 1, wherein the gold salt and platinum salt are used in such a way that an amount of Pt is 7 to 25% of an amount of Au.

3. A labeling agent for an immunoassay comprising the alloyed Au/Pt composite colloid according to claim 1 or 2

4. An antibody or antigen labeled with the alloyed Au/Pt composite colloid according to claim 1 or 2.

5. A method for producing an alloyed Au/Pt composite colloid comprising mixing a gold salt and a platinum salt with at least one reducing agent selected from the group consisting of an amino acid and a derivative thereof, an oligopeptide and a derivative thereof, and an amino sugar in the presence of an alkali, thereby reducing the gold salt and platinum salt.
